# EUROPEAN PATENT SPECIFICATION

(11) **EP 2 912 584 B1**
(45) Date of publication and mention of the grant of the patent: **07.10.2020**
(21) Application number: 13821154.5
(22) Date of filing: 17.10.2013
(51) Int. Cl.: G16H 50/30, G16H 50/70

(54) **COMBINED USE OF CLINICAL RISK FACTORS AND MOLECULAR MARKERS FOR THROMBOSIS FOR CLINICAL DECISION SUPPORT**
KOMBINIERTE VERWENDUNG VON KLINISCHEN RISIKOFAKTOREN UND MOLEKULAREN MARKER FÜR THROMBOSE FÜR KLINISCHE ENTSCHEIDUNGSUNTERSTÜTZUNG
UTILISATION COMBINÉE DE FACTEURS DE RISQUE CLINIQUE ET DE MARQUEURS MOLÉCULAIRES DE THROMBOSE POUR AIDE À LA DÉCISION CLINIQUE

(30) Priority: 25.10.2012 US 201261718242 P
(43) Date of publication of application: 02.09.2015
(73) Proprietor: Koninklijke Philips N.V., 5656 AG Eindhoven (NL)
(72) Inventor: BAKKER, Bart Jacob, NL-5656 AE Eindhoven (NL); VAN OOIJEN, Hendrik Jan, NL-5656 AE Eindhoven (NL); VAN DEN HAM, René, NL-5656 AE Eindhoven (NL)
(74) Representative: Philips Intellectual Property & Standards
(86) International application number: PCT/IB2013/059424
(87) International publication number: WO 2014/064585

(56) References cited:
- US-A1- 2009 089 079
- US-A1- 2009 089 079
- S. PENCO ET AL: "Assessment of the Role of Genetic Polymorphism in Venous Thrombosis Through Artificial Neural Networks", ANNALS OF HUMAN GENETICS, vol. 69, no. 6, 1 November 2005 (2005-11-01), pages 693-706, XP055106644, ISSN: 0003-4800, DOI: 10.1111/j.1529-8817.2005.00206.x
- H. G. DE HAAN ET AL: "Multiple SNP testing improves risk prediction of first venous thrombosis", BLOOD, vol. 120, no. 3, 14 May 2012 (2012-05-14), pages 656-663, XP055105755, ISSN: 0006-4971, DOI: 10.1182/blood-2011-12-397752
- NOVIS S J ET AL: "Prevention of thromboembolic events in surgical patients through the creation and implementation of a computerized risk assessment program", JOURNAL OF VASCULAR SURGERY, C.V. MOSBY CO., ST. LOUIS, MO, US, vol. 51, no. 3, 1 March 2010 (2010-03-01), pages 648-654, XP026931275, ISSN: 0741-5214, DOI: 10.1016/J.JVS.2009.08.097 [retrieved on 2010-03-02]
- FRITS R ROSENDAAL: "Venous Thrombosis: The Role of Genes, Environment, and Behavior Ham-Wasserman Lecture", HEMATOLOGY, vol. 2005, no. 1, 1 January 2005 (2005-01-01), pages 1-12, XP055105734,
- S. Penco ET AL: "Assessment of the Role of Genetic Polymorphism in Venous Thrombosis Through Artificial Neural Networks", Annals of Human Genetics, vol. 69, no. 6, 1 November 2005 (2005-11-01), pages 693-706, XP055106644, ISSN: 0003-4800, DOI: 10.1111/j.1529-8817.2005.00206.x
- H. G. De Haan ET AL: "Multiple SNP testing improves risk prediction of first venous thrombosis", Blood, vol. 120, no. 3, 14 May 2012 (2012-05-14), pages 656-663, XP055105755, ISSN: 0006-4971, DOI: 10.1182/blood-2011-12-397752
- NOVIS S J ET AL: "Prevention of thromboembolic events in surgical patients through the creation and implementation of a computerized risk assessment program", JOURNAL OF VASCULAR SURGERY, C.V. MOSBY CO., ST. LOUIS, MO, US, vol. 51, no. 3, 1 March 2010 (2010-03-01), pages 648-654, XP026931275, ISSN: 0741-5214, DOI: 10.1016/J.JVS.2009.08.097 [retrieved on 2010-03-02]
- Frits R Rosendaal: "Venous Thrombosis: The Role of Genes, Environment, and Behavior Ham-Wasserman Lecture", Hematology, vol. 2005, no. 1, 1 January 2005 (2005-01-01), pages 1-12, XP055105734,
- Juan Carlos Souto: "Predicting individual risk of venous thrombosis", , 19 July 2012 (2012-07-19), XP055436146, Retrieved from the Internet: URL:http://www.bloodjournal.org/content/12 0/3/500.full.pdf [retrieved on 2017-12-18]
- Ron Ascherson: "antiphospholipid antibodies", , 12 October 2012 (2012-10-12), XP055436176, Retrieved from the Internet: URL:https://web.archive.org/web/2012101200 4932/http://www.arthritis.co.za/saraa/sara aantiphospholipid.htm [retrieved on 2017-12-18]
- Neil Zakai ET AL: "Summary : The Longitudinal Investigation of Thromboembolism Etiology", Thrombosis and Haemostasis, vol. 104, no. 08, 1 January 2010 (2010-01-01), pages 207-212, XP055615765, DE ISSN: 0340-6245, DOI: 10.1160/TH09-10-0693

## Description

### FIELD OF THE INVENTION

The invention relates to the field of clinical decision support where an estimation value of thrombosis risk of a patient is calculated based on patient-specific input features.

### BACKGROUND OF THE INVENTION

Computer-based clinical decision support systems (CDSSs) are defined as "any software designed to directly aid in clinical decision making in which characteristics of individual patients are matched to a computerized knowledge base for the purpose of generating patient-specific assessments or recommendations that are then presented to clinicians for consideration and decision making". Clinical decision support systems have been promoted for their potential to improve the quality of health care by supporting clinical decision making.

Deep vein thrombosis is a wide spread problem in the western world. Large portions of the population are at increased risk of thrombosis, e.g. the elderly, people who travel, and patients that undergo orthopedic surgery. People at risk can be put on preventive anticoagulant treatment, but the risk of bleeding (1-3% per year), and issues of cost and inconvenience speak against this. It would therefore be desirable to have a more patient-specific measure to estimate the personal thrombosis risk and facilitate an informed choice on whether or not to treat. Unfortunately, with current clinical screening techniques and available methodologies, high risk individuals, which should receive anticoagulants, are not easily recognized and events are not accurately predicted. One of the main reasons that this continues to be the case is that the vast majority of patients who suffer from thrombosis, those without obvious genetic defects, have blood coagulation systems that are not clinically identified as abnormal by routine screening tools and factor assays. Identification of individuals who are at risk for venous thrombosis is an area of research that could benefit from innovative technical methods.

Uncertainty about the patient specific risk of thrombosis causes unnecessary thromboses in patients at high risk (of thrombosis) who do not receive anticoagulant treatment. On the other hand, this uncertainty can result in bleeding in patients at relatively low risk who do receive unnecessary anticoagulant treatment. Most conventional clinical decision support systems are adapted to estimate thrombosis risk based on a number of clinical risk factors. A number of clinical risk factors such as immobility and contraceptive use have been identified (for patients without obvious genetic defects), but these are not sufficient for screening purposes. In practice, as described in Durieux et al.: "A Clinical Decision Support System for Prevention of Venous Thromboembolism", guidelines based on clinical risk factors are used.

A conceptually different world compared to clinical risk factors based stratification is disclosed in US 2009/0298103 A1 where a single simulation of a protein based measurement, i.e. the thrombin generation assay, is linked to thrombotic risk.

US2009/0089079 discloses a method for determining whether to issue an alert to consider prophylaxis with respect to a thrombosis risk of a subject based on clinical risk factors of the subject.

Penco et al. (2005), Assessment of the role of genetic polymorphism in venous thrombosis through artificial neural networks, Annals of Human Genetics 69:693-706, disclose a study exploring the association between venous thrombotic events and a multilocus genotype exploiting the properties of Artificial Neural Networks.

Haan et al. (2012), Multiple SNP testing improves risk prediction of first venous thrombosis, Blood 120:656-663, disclose a method for predicting the risk of first venous thrombosis based on a combination of genetic markers and clinical risk factors.

Novis et al. (2010), Prevention of thromboembolic events in surgical patients through the creation and implementation of a computerized risk assessment program, Journal of Vascular Surgery 51:648-654, disclose a method for preventing the development of thrombosis based on clinical risk factors.

Other prior art documents considered during examination include:
FRITS R ROSENDAAL: "Venous Thrombosis: The Role of Genes, Environment, and Behavior Ham-Wasserman Lecture", HEMATOLOGY, vol. 2005, no. 1, 1 January 2005, pages 1-12.

Juan Carlos Souto: "Predicting individual risk of venous thrombosis", 19 July 2012, http://www.bloodjoumal.org/content/120/3/500.full.pdf.

Ron Ascherson: "antiphospholipid antibodies", 12 October 2012 (2012-10-12), https://web.archive.org/web/20121012004932/http://www.arthritis.co.za/saraa/saraaantiphosp hoiipid.htm.

However the above approaches are not sufficiently specific for screening of thrombosis because the number of patients wrongfully classified is still high using the currently available methods.

### SUMMARY OF THE INVENTION

It is an object of the invention to provide a clinical decision support system with increased accuracy for of person specific thrombosis risk estimation.

This object is achieved by an apparatus as claimed in claim 1, a method as claimed in claim 6, and by a computer program product as claimed in claim 9.

Accordingly, two conceptually different worlds of clinical risk factors and molecular markers are combined. This proposed combination is non-trivial to make and requires a significant effort of machine learning and data driven approaches. The smallest set of risk factors and protein concentrations that together have an optimal predictive value for thrombosis risk are selected and a numerical algorithm is created that translates the numerical value of the chosen factors and concentrations to a single numerical value specifying thrombotic risk. Thereby, accuracy of person specific thrombosis risk estimation can be increased substantially, especially within the increased risk subgroup of patients with at least one known clinical risk factor present. This subgroup involves (among others) patients that are hospitalized, are pregnant or are (start) using oral contraceptives and thus receive attention of a physician. In this context, the proposed solution helps the physician to stratify the patients that are treated or examined for conditions that are known to increase thrombosis risk, into high and low risk categories. Specifically, the proposed solution may be used to decide, per patient, whether or not to administer anticoagulant treatment based on estimated thrombosis risk.

The term "molecular marker" is intended here to include any use of the presence or concentration of a biomolecule or part of a biomolecule, e.g., a protein or a polynucleid acid as an indicator of a patient phenotype. Such presence or concentration may be measured directly in e.g. a blood or tissue sample, or as a (possibly dynamic) measurement of the molecule in a functional test like real-time quantitative polymerase chain reaction (PCR) or the thrombin generation assay

The plurality of protein concentrations includes a concentration of coagulation protein FVIII in blood, a concentration of coagulation protein FXI in blood, and a concentration of coagulation protein TFPI in blood. Based on patient datasets obtained from a clinical study, these types of protein concentrations have turned out to serve as reliable indicators of thrombotic risk.

The plurality of clinical risk factors includes immobilization within the last three months from wearing a plaster case, from extended bed rest at home for at least four days, or from hospitalization, surgery within the last month, a history of venous thrombosis in at least one parent, brother or sister, pregnancy or puerperium within the last three months, current use of estrogens in oral contraceptives or hormone replacement therapy, and obesity, indicated by a body mass index over 30. These clinical risk factors have been selected based on the above patient datasets of the specific clinical study as most reliable in combination with the above specific protein concentrations.

The estimation value of thrombotic risk may be compared with a predetermined threshold value in order to classify the estimation value based on the comparison result. Thereby, decision making by a clinician can be supported by classifying patients into groups of predetermined risk levels, e.g., high and low thrombotic risk.

A user may be allowed to input or disable the predetermined threshold value. Thereby, the decision support mechanism can be adapted based on the needs of the user (i.e. clinician).

An optimization mechanism may be provided for applying a learning process through an optimization procedure based on a dataset stored in a database so as to minimize a prediction error. This allows continuous adaptation of the clinical decision support mechanism to new datasets of new patients or to specific datasets of individual patients.

The dataset may be divided into a training set, a validation set and a test set, wherein the training set and the validation set may be used to select a type of machine learning function and a set of model parameters used for optimizing classifiers, wherein the optimized classifiers may be used for obtaining the patient-specific input features, and wherein the test set may be used for monitoring the estimation value for patients of the test set based on the obtained input features. This measure allows specific trimming of the input features of the clinical decision support system to a data set obtained from a specific group of patients to thereby further enhance reliability of risk estimation.

The processor may be adapted to calculate a deep vein thrombosis (DVT) risk score, representing an estimation value of thrombosis risk of a patient, based on clinical risk factors, single nucleotide polymorphisms (SNPs) and protein levels. This DVT risk score shows significant improvement in terms of sensitivity/specificity over known methods that calculate a DVT risk score without protein levels.

It is noted that the apparatus may be implemented as a discrete hardware circuitry with discrete hardware components, as an integrated chip, as an arrangement of chip modules, or as a signal processing device or chip controlled by a software routine or program stored in a memory, written on a computer readable medium, or downloaded from a network, such as the Internet.

It shall be understood that the apparatus of claim 1, the method of claim 6, and the computer program product of claim 9 have similar and/or identical preferred embodiments, in particular, as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the drawings:
Fig. 1 shows a schematic block diagram of a clinical decision support system according to various embodiments;
Fig. 2 shows a flow diagram of a risk estimation procedure according to a first embodiment;
Fig. 3 shows a flow diagram of a classifier optimization procedure according to a second embodiment;
Fig. 4 shows a schematic representation of a user interface according to a third embodiment;
Figs. 5A and 5B respectively show a receiver operator curve (ROC) plus 95% confidence interval for thrombosis predicted by a support vector machine with only clinical risk factors as input resulting and a ROC curve plus 95% confidence interval for thrombosis predicted by a classifier with clinical risk factors and protein concentrations as inputs; and
Figs. 6A and 6B respectively show a ROC plus 95% confidence interval for thrombosis, predicted within the subgroup of patients with one or more known clinical risk factors present, by a support vector machine with only clinical risk factors as input and a ROC curve plus 95% confidence interval for thrombosis predicted by a classifier with clinical risk factors and protein concentrations as inputs.

### DETAILED DESCRIPTION OF EMBODIMENTS

Embodiments of the invention as defined by the independent claims are now described based on a computerized clinical decision support system for predicting thrombosis risk based on a combined consideration of clinical risk factors and molecular markers, i.e., protein concentrations.

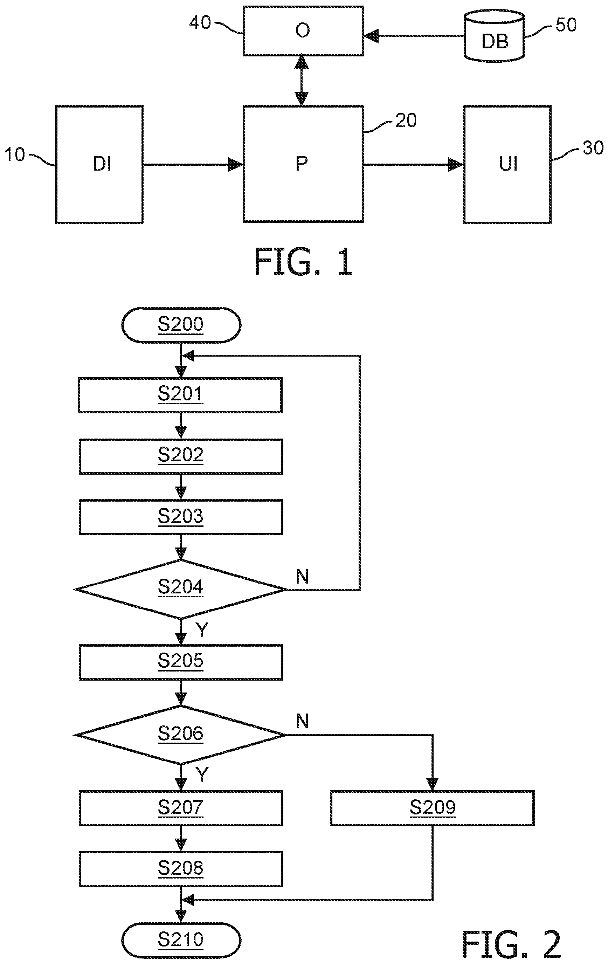

Fig. 1 shows a schematic block diagram of a clinical decision support system according to various embodiments, which involves a clinical decision support algorithm and/or software. It comprises data interface (DI) 10 where information about a specific patient is made available to the system, a processor (P) 20 which applies an interpretative algorithm and a user interface (UI) 30 which makes the interpretation of the calculated data available to a user, e.g., a clinician. Furthermore, an optional optimization system may be provided for optimizing classifiers so as to provide a good trade-off between good prediction accuracy and conciseness of the set of input features or parameters for the clinical decision support algorithm. The optimization system comprises an optimization unit (O) 40 which may be based on a separate processor running an optimization software or based on a separate software routine controlling the processor 20. The optimization unit 40 retrieves data required for optimization from a database (DB) 50.

The data interface 10 may be a classical user interface for allowing interaction between a user and the clinical decision support system, or a direct link to a central computer database or electronic patient record. In either case, the data interface 10 is adapted to collect at least some of the following input features on a patient at the date on which the clinical decision support system is used to assess thrombosis risk:
1. immobilization (plaster cast, extended bed rest at home for at least 4 days, hospitalization) within the last three months (e.g. "1" for true, "0" for false);
2. surgery within the last month (e.g. "1" for true, "0" for false);
3. family history of venous thrombosis (considered positive if at least one parent, brother, or sister experienced venous thrombosis (e.g. "1" for true, "0" for false));
4. pregnancy or puerperium within the last three months (e.g. "1" for true, "0" for false);
5. current use of estrogens (oral contraceptives or hormone replacement therapy (e.g. "1" for true, "0" for false));
6. obesity (body mass index over 30 (e.g. "1" for true, "0" for false));
7. concentration (U/mL) of the coagulation protein FVIII in blood;
8. concentration (U/mL) of the coagulation protein FXI in blood; and
9. concentration (ng/ml) of the coagulation protein TFPI in blood.

In the above, the units and possible numerical values for each input feature are given for clarity, but the choice of specific units is not essential.

Based on at least some of the above input features, the processor 20 calculates a numerical function of the above list of numerical inputs by applying the clinical decision support algorithm. This numerical function returns a number, i.e. risk score (R), between zero and one, where zero is the lowest possible thrombosis risk indication and one is the highest. This numerical output may be shown directly on the user interface 30 and/or may be compared to a threshold (T) between zero and one. If the risk score exceeds the threshold T, anti-coagulant therapy is indicated for the patient for whom the values have been entered into the calculation. Otherwise, preventive anti-coagulation therapy is indicated as not advisable. The choice of T, which can be set as a fixed value in the system or tuned by the user at the user interface 30, determines the balance between sensitivity and specificity of the clinical decision support system. Low values for T will infer a bias towards the indication of high risk, which leads to few false negatives (high sensitivity) but increases the number of false positives (low specificity or overtreatment). High values for T give the opposite effect and tends to undertreatment. The specific choice of T is the responsibility of the user, e.g. clinician, and may be the subject of a clinical study, but is not further dicussed here.

The clinical decision support system may be implemented as a software application on a computer (system) that can be accessed by a clinician who needs to make a decision about patients' anticoagulation treatment. Optionally, the software application of the clinical decision support system may be integrated (e.g. as a plug-in) in an existing hospital information management system.

The interpretative clinical decision support algorithm may be a complex mathematical function that takes numerical (or Boolean) values for the above nine input features as input, uses these in a series of non-linear calculations and returns a numerical value between zero and one, where higher values represent a higher risk of thrombosis. The numerical function consists of one or a combination of classifier functions that are common in the field of machine learning, such as neural network functions or support vector machines or Bayesian network. These classifiers are optimized by the optimization unit 40 based on the database 50 of subjects, i.e. thrombosis patients and healthy controls for whom numerical values for the aforementioned nine input features are available. Optimization of the optimization unit 40 involves tuning the parameters of the classifier functions in such a way that the correlation between calculated risk score on the subjects in the database and recorded occurrence of thrombosis is maximized. The optimization process constitutes a significant effort that requires a strong experience in and understanding of the field of machine learning and numerical optimization. The process is further strongly dependent on the quality of the underlying database 50.

Fig. 2 shows a flow diagram of a thrombosis risk estimation process according to a first embodiment. After the start of the procedure in step S200, the data interface 10 accesses in step S201 the hospitals electronic patient record (EPR), if present, and reads out the nine patient features 1 - 9 that were listed above. Optionally, the user may be requested or allowed to manually enter, e.g. via the user interface 30, numerical values for patient features that are not available from the EPR. Then, in step S202, the data interface 10 checks the entered values for the right numerical format and an error message can be generated if the input format does not match with the required format. In case of a wrong format, the data is converted in step S203 to the numerical formats indicated in the above list, if necessary. Additionally, the user interface 30 may allow the user either to enter a numerical value for the threshold T between zero and one, or to disable the threshold.

Then, in step S204, the procedure checks whether risk calculation has been requested by the user (e.g. through clicking on a respective button at the user interface 30). If not, the systems repeats the above steps S201 to S203 to allow an update of the input features or simply repeats step S204 until risk calculation is requested. I.e., the "No" branch arrow of step S204 can simply point back to the top of step S204 and needs not go back to step S201. If the request is detected in step S204, clinical decision support algorithm is called in step S205 (e.g. by the processor 20) to calculate a risk score based on the input features gathered in the previous steps.

In the subsequent step S206, it is checked if the threshold (T) has been enabled. If not, the procedure branches to step S209 and the calculated risk score is shown as a number or another graphical representation e.g. on a computer screen or other output medium of the user interface 30 before the procedure ends in step S210. Otherwise, if the procedure detects in step S206 that the threshold has not been disabled, the risk score is compared in step S207 to the threshold and classified based on the result of comparison. Finally, in step S208 a classification of 'high thrombosis risk' or 'low thrombosis risk' is made visible e.g. on the screen of the user interface 30 dependent on whether the risk score is higher or lower than the threshold. Optionally, a numerical and/or graphical comparison between the threshold value and the risk score should be shown along with the classification.

According to a modification of the first embodiment, the risk score could be calculated continously (instead of upon request). This could also be done with some of the missing input parameters. In that case, a range of possible risk scores (e.g., indicated by a minimum risk estimation and maximum risk estimation) is provided as output, e.g., based on an uncertainty in the calculation.

In the following, an optimization of the clinical decision support algorithm is described based on a second embodiment.

The required data set of the database 50 may be derived from a data collection based on an extensive questionnaire on many potential risk factors for venous thrombosis. More specifically, the data collection may involve information (e.g. clinical risk factors) obtained from a questionnaire and clinical assays (e.g. activity or antigen-based assays of protein concentrations) as described in the respective assay protocols.

Machine learning methods are black box methods that exploit the patterns that may be hidden in the numerical values of the data to predict an output. Each method constructs a mathematical function that takes observed quantities (like protein concentrations) and qualities (like immobilization) as inputs, and produces an output that predicts a certain desired feature. Such a function is defined through its structure (e.g. a neural network function) and the numerical value of the function parameters (e.g. the weights in a neural network). The combination of function structure, parameter values and numerical inputs produce an output feature which may be binary (e.g. thrombosis vs. no thrombosis), or continuous (e.g. probability of thrombosis). The specific type of method that is used in the second embodiment is the support vector machine (SVM), an often used method in the field of machine learning (see e.g. Cristianini et al.: "An Introduction to Support Vector Machines and Other Kernel-based Learning Methods", Cambridge University Press, 2000 for more details). A hidden pattern is 'learned' directly from the data, generally without concern for the identity (e.g. biological meaning) of the various inputs. Learning proceeds through an optimization procedure, where the prediction error (i.e. some numerical measure of the discrepancy between predicted model output and observations) is minimized. There are many optimization or error minimization routines which all involve the variation of the mathematical function's parameters to find that set of parameter values that produces the lowest prediction error. A wide literature exists on machine learning techniques and optimization methods. For a more in-depth view, it is referred to Kuncheva: "Combining Pattern Classifiers: Methods and Algorithms", Wiley-Blackwell 2004.

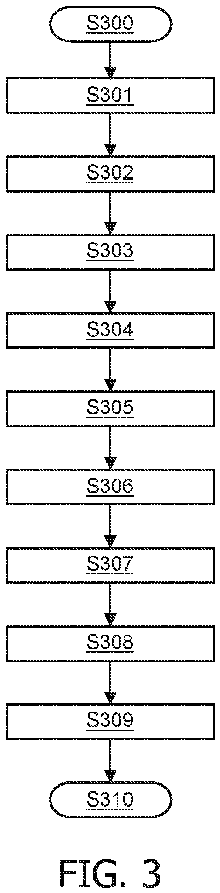

Fig. 3 shows a flow diagram of an optimization process according to a second embodiment.

A classifier is a specific class of black box model, the output of which is the class or label of a data element, where each element is described by a number of numerical features. The data elements in the present embodiments are human subjects for whom a number of clinical features are known through measurement or anamnesis. The class is binary: thrombosis patient or control subject. The classifier is trained on the dataset of the database 50 which contains each participant's numerical features and the corresponding label.

After the start of the optimization procedure in step S300, the dataset of the database 50 is divided in step S301 into three equally sized sets, called training set, validation set and test set, each containing the same ratio of cases to controls. In step S302, the training set is used for training or parameter tuning, i.e. search for that set of parameter values that minimizes the prediction, or in this case classification error. Most machine learning methods suffer from so-called 'overfitting', where the method's performance on the training set is much better than its performance on new data that has not been used for training. Therefore, in step S303, a separate validation set is used to test whether such over-fitting occurs. The combination of training and validation data allows to find that type of machine learning function and choice of model parameters that is able to grasp the true pattern that hides in the (training) data, yet is still sufficiently general to predict well on the separate validation data and thus on future data as well. The thus optimized classifiers are used in step S304 to make a prediction on each of the patients in the test set, which has remained unused throughout the foregoing optimization steps. The quality of this prediction (e.g. in terms of sensitivity and specificity) is the final test of the validity of the selected classifier. The test set is selected at random to obtain solid statistics.

The steps S301 to S303 described the selection of an optimal classifier based on a train and validation subset of a database. Through permutation of the subjects in the train and the validation set (swapping patients between the two sets) in step S305 it is possible to create an ensemble of classifiers, each classifier corresponding to one specific permutation of train and validation subjects. Such an ensemble is used as a voting system. This means that each classifier in the ensemble assigns a label to the same object, e.g. 'control subject' or 'thrombosis patient'. The label that turns up most often is assumed to be the correct one, and the fraction of votes that support this label are used as a confidence score: if all classifiers in the ensemble vote for thrombosis, it is 100% sure that the participant will get thrombosis, whereas a fifty-fifty distribution of the votes makes the classification no better than a coin flip. The risk score (R) is compared to a threshold (T), where a score that exceeds the threshold indicates a case and a score below the threshold indicates a control subject.

When the optimal classifier on the complete set of features has been found in step S305, the relative importance of each input feature in the classifier is analyzed in step S306. The selected subjects in the train and validation set are now used to select those features that contribute most to a correct classification. To achieve this, the following input reduction procedure is executed in step S306 for each of the optimized classifiers:
For each input feature i to the classifier
Remove input feature i
Re-optimize the reduced classifier on the train set
Calculate the resulting prediction error on the train set
Restore input feature i
Permanently remove the input feature with the lowest prediction error
Repeat from start until only one input feature is left.

As the number of input features in the classifier reduces, the prediction error rises. Thus, there is always a trade-off between good prediction ability and conciseness of the set of input features used. The above reduction procedure is used to deduce a selection of overall most predictive features. It is performed for each aforementioned (random) division of the complete database into a train, validation and test set. In step S307, for each division, the classifier is reduced to ten input features, and each remaining input feature is marked. Then, in step S309, the number of times each input feature remains in the 'top ten' is counted and this count is used to rank the input features from most predictive (part of the top ten most often) to least predictive. Finally, the most predictive input features are used for risk calculation in the clinical decision support algorithm of the processor 20 and the procedure ends in step S310.

Hence, the optimization procedure of the second embodiment can be used to regularly update the clinical decision support algorithm of the processor 20 based on new patient data in the database 50.

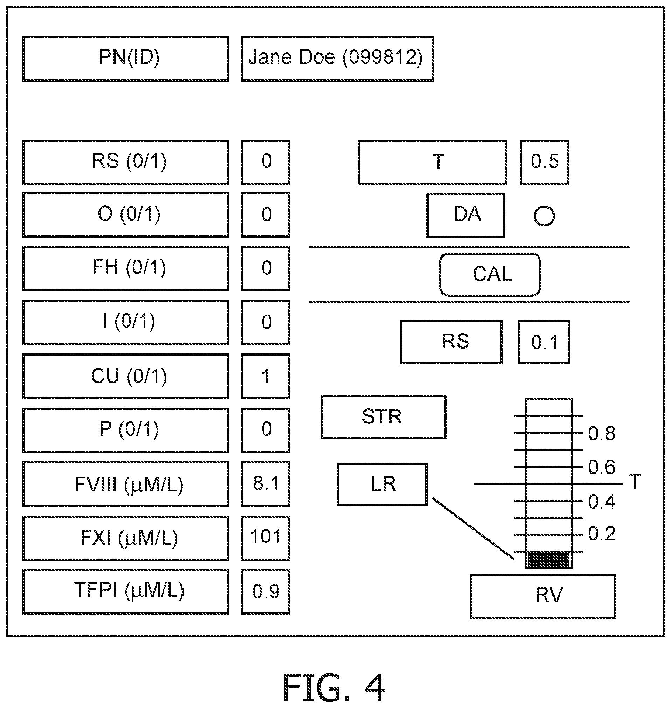

Fig. 4 shows a schematic representation of a front view of the user interface 30 of Fig. 1. In the left portion, the patient name (PN) and its identification number (ID) is indicated as "Jane Doe" and "099812". Below this information, nine input features are designated and their actual binary values ("0" or "1") of the above patient are indicated on the right side beneath the designation. The first six input features are the clinical risk factors indicating recent surgery (RS), obesity (O), family history (FH), Immobility (I), contraceptive use (CU) and pregnancy (P). The last three input features are the concentration levels of coagulation proteins Factor VIII (FVIII), Factor XI (FXI) and tissue factor pathway inhibitor (TFPI). On the right portion, the currently set threshold level (T) is indicated (i.e. 0.5) and the status of the disabling (DA) function is indicated below. This may be simply a light or color indicator. Further below, a button (CAL) for activating or triggering a risk calculation by the processor 20 is shown. Below this button, a numerical indication of the calculated risk score (RS) (i.e. 0.12) is provided and further below a graphical visualization (RV) of this risk score on a risk scale in relation to the threshold T is shown as a stratification (STR). The bar which indicates the current risk score on the risk scale is qualified as low risk (LR). This visualization together with the other output information and input functions on the user interface 30 allows quick assessment by the user, i.e. clinician, and provides enhanced support for treatment decision.

The following example is presented by way of illustration of the present invention, which is defined by the independent claims, and are not intended to limit the present invention and the embodiments provided herein in any way.

In a first example which relates to thrombosis risk classification, the second embodiment explained above was applied to a clinical study of -500 thrombosis patients and -500 healthy controls, and showed that the proposed solution leads to significantly better results in terms of estimation accuracy than a 'conventional' approach based on clinical risk factors alone. An ensemble of support vector machines was used on the LeidenThrombophilia Study (LETS) (as described for example in van der Meer et al.: "The LeidenThrombophilia Study (LETS)", Thromb Haemost. 1997;78(1):631-5) in order to find a combination of known biomarkers that is able to distinguish thrombosis patients from healthy controls. Focus was directed at two different types of patient features, i.e. coagulation protein concentrations in blood and clinical risk factors that are known to relate to thrombosis. It could be shown that the predictive power of clinical risk factors alone, either as a simple risk factor count or used in a machine learning approach, can be improved by incorporation of measured coagulation protein concentrations.

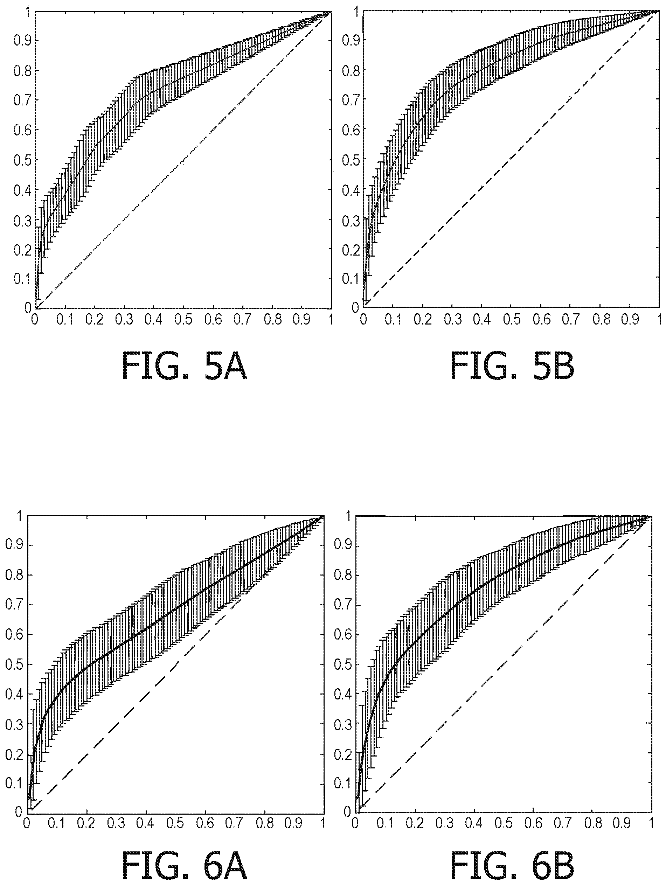

Figs. 5A and 5B show respective diagrams with a receiver operator curve (ROC) plus 95% confidence interval for thrombosis predicted by a support vector machine with only clinical risk factors as input resulting in an area under the ROC curve (AUC) of 0.72 (0.68 - 0.77) (Fig. 5A) and a ROC curve plus 95% confidence interval for thrombosis predicted by a classifier with clinical risk factors and protein concentrations as inputs resulting in an AUC of 0.78 (0.74 - 0.83) (Fig. 5B). The ROC curves plot the true positive rate (vertical axis) against the false positive rate (horizontal axis) for different threshold values. The area under the ROC curve (AUC) is used as a measure for the quality of the classifier ensemble. As can be gathered from Figs. 5A and 5B, the combination of both types of features gives a significantly better classification (i.e. AUC of 0.78 vs. 0.72, p<0.001).

A second example relates to input feature reduction. In the study, the determined most influential protein in thrombosis classification was coagulation factor VIII, followed by factor XI and TFPI (cf. Table 1 below). Classification with all clinical risk factors (for which no measurement is necessary) and these three protein concentrations achieves almost equivalent classification at AUC of 0.77. The improvement is especially clear in the increased risk population, here defined as those subjects showing one or more known clinical risk factors.

Figs. 6A and 6B show the ROC plus 95% confidence interval for thrombosis, predicted within the subgroup of patients with one or more known clinical risk factors present, by a support vector machine with only clinical risk factors as input resulting in an AUC of 0.67 (0.60 - 0.75) (Fig. 6A), and a ROC curve plus 95% confidence interval for thrombosis predicted by a classifier with clinical risk factors and protein concentrations as inputs resulting in an AUC of 0.75 (0.69 - 0.81) (Fig. 6B).

As can be gathered from Figs. 6A and 6B, the use of the three protein concentration values allows a further stratification of this risk group with an ROC score of 0.75 versus 0.67 based on the use of clinical risk factors alone (number of co-occurring factors or knowledge of which factor is present).

Table 1 shows a list of classifier features, sorted by the percentage of classifiers (based on different random choices of validation set) that retain the feature in the 10 features that are pruned last.

**Table 1**

| Rank | Feature name | Classifiers (%) | Rank | Feature name | Classifiers(%) |
|---|---|---|---|---|---|
| 1 | F8 | 100 | 14 | Obesity | 23 |
| 2 | Contraceptive use | 100 | 15 | Protein C | 21 |
| 3 | Immobility | 100 | 16 | F9 | 17 |
| 4 | Surgery | 100 | 17 | Protein S | 14 |
| 5 | Family history of thrombosis | 89 | 18 | ZPI | 12 |
| 6 | F11 | 80 | 19 | F13 | 8 |
| 7 | Pregnancy/puerperium | 74 | 20 | F2 | 7 |
| 8 | TFPI | 74 | 21 | AT | 5 |
| 9 | C4BP | 50 | 22 | PCI | 2 |
| 10 | Protein Z | 37 | 23 | F10 | 1 |
| 11 | F12 | 37 | 24 | F7 | 0 |
| 12 | Fibrinogen | 26 | 25 | F5 | 0 |
| 13 | TAFI | 24 | | | |

The risk of deep vein thrombosis has been evaluated by using information from the MEGA (Multiple Environment and Genetic Assessment of risk factors for venous thrombosis) study and the Leiden Thrombophilia Study (LETS). Both are case-control studies that were set up to identify risk factors for venous thrombosis that have been performed in the Netherlands (Blom, 2005, van der Meer FJ, Koster T, Vandenbroucke JP, Briët E, 1997). A plethora of variables, ranging from coagulation protein levels to environmental thrombotic risk factors and genetic thrombophilia has been taken from patients with venous thrombosis and controls. For the purpose of this study, a neural networks approach (see e.g. Kuncheva, 2004) has been used in the MEGA study to estimate potential risk factors for Deep Vein Thrombosis (DVT) and their predictive value in one integrated approach. The identified combinatory risk score is validated in an internal cross-validation on the MEGA study and in an independent validation on the LETS study.

It has been shown in the past that a combination of clinical risk factors and single nucleotide polymorphisms (SNPs) allowed discrimination between high and low risk patients with an area under the Receiver Operating Characteristic (ROC) curve (AUC) of 0.82 on MEGA and 0.77 on LETS. It is now shown that through the addition of protein levels as predictive factors a significant further increase in predictive accuracy can be achieved as quantified in the AUCs of 0.87 and 0.81 respectively.

Further, four clinical risk factors that were not available for the initial study are now considered: immobilization because of plaster cast, leg injury in the past 3 months, cancer in the period from five years before to six month after the index date and travel for more than four hours in the past 2 months. The other considered risk factors were part of the initial study as well: immobilization because of extended bed rest at home for at least 4 days, hospitalization), surgery, a family history of venous thrombosis (considered positive if at least 1 parent, brother, or sister experienced venous thrombosis, pregnancy or puerperium within 3 months before the index date, or use of estrogens (oral contraceptives or hormone replacement therapy) at the index date and the presence of obesity, determined as a body mass index of 30 kg/m2 or higher).

Next to the data from the questionnaire and measured protein levels, data was available on the presence of five genetic aspects, i.e. blood group and four single nucleotide polymorphisms (SNPs) in F2 (G20210A), Fibrinogen (rs no 2066865), F11 (rs no 2036914) and F5 (FV Leiden; rs no 6025). The data further included the number of alleles that were affected per SNP.

The considered protein levels are a subset of the proteins that were included before (because of a more limited set of measurements performed in the MEGA study). They are: anti-thrombin (AT), prothrombin (factor II), factor 7 (FVII), FVIII, FIX, FX, FXI, fibrinogen and protein C (all activity measurements) and protein S (antigen measurement).

Cross-validation results on MEGA. Neural networks based risk scores that predict risk based on clinical risk factors, genetic effects and protein levels to risk scores based on clinical risk factors and genetic effects (without protein levels) and clinical risk scores based only on clinical risk factors were considered. The comparison is performed on the MEGA study, but otherwise in the same cross-validation setup and with the same methods as described in the initial study. The corresponding AUC's are 0.87, 0.83 and 0.78, i.e. each addition improves the accuracy of the risk score; all improvements are significant (p<0.01 in a paired t-test).

The LETS study includes four less clinical risk factors than the MEGA study, as described above with respect to the clinical risk factors. The cross-validation as performed in the previous paragraph has been repeated without these four risk factors and under the exclusion of cancer patients, who had been excluded from the LETS study as well. The AUCs on the reduced MEGA study are 0.84, 0.80 and 0.74, in the same order as in the last paragraph. Next, for each of the selections of input features (clinical risk factors with/without genetic effects with/without protein levels) one risk score on the reduced MEGA study (without divisions into train and test set as would be necessary in a cross-validation) was derived and applied this risk score without adaptation to the individuals of the LETS study. The resulting AUCs were 0.82, 0.79 and 0.74, showing that the proposed risk score can be applied on an independent study with little loss of performance, and the improvement due to the proposed inclusion of protein levels holds in an external validation.

The same methods are used in a cross-validation study within the MEGA subpopulation of individuals with one or more of the aforementioned clinical risk factors present (this was done for the LETS study in the initial filing as well). The resulting AUCs were 0.86, 0.81 and 0.76 for the three scoring methods, again with lower scores for scores that consider fewer input features.

Following the same methods as described above, the importance of all features that were used as inputs to the neural networks that provide the risk score were ranked. The results are shown in Table 2. The results overlap partially with the earlier results: F8 is still by far the most predictive protein and contraceptive use, surgery, immobility and family history still score high. TFPI has not been measured in MEGA and does therefore not appear in the ranking. F11 scores much lower than before.

**Table 2**

| Rank | Feature name | Top 10 (%) | Rank | Feature name | Top 10 (%) |
|---|---|---|---|---|---|
| 1 | F8 | 100 | 14 | F11 SNP | 15 |
| 2 | Oral contraceptive use | 100 | 15 | Prothrombin | 13 |
| 3 | Leg injury | 100 | 16 | Protein C | 13 |
| 4 | FV Leiden | 100 | 17 | Pregnancy | 12 |
| 5 | Surgery | 88 | 18 | Blood type | 12 |
| 6 | Immobility (hospitalization) | 87 | 19 | AT | 10 |
| 7 | Family history | 85 | 20 | FIX | 9 |
| 8 | Protein S | 68 | 21 | FXI | 8 |
| 9 | Fibrinogen SNP | 54 | 22 | Plaster cast | 8 |
| 10 | Immobility (at home) | 38 | 23 | Cancer | 5 |
| 11 | Obesity | 22 | 24 | FVII | 5 |
| 12 | FX | 21 | 25 | Fibrinogen | 5 |
| 13 | F2 SNP | 17 | 26 | Travel | 3 |

Cross-validation on MEGA with a risk score based on all clinical risk factors, one SNP (FV Leiden) and the protein level of FVIII provides an accuracy that is only a little reduced (AUC=0.85 vs 0.87). Further addition of the SNP in fibrinogen and the protein levels of protein S and FX increase the AUC to 0.86.

As explained above a DVT risk score based on clinical risk factors, SNPs and protein levels shows significant improvement in terms of sensitivity/specificity over known methods without protein levels in an evaluation on the MEGA study.

To summarize, an apparatus and method have been described for clinical decision support to identify patients at high risk of thrombosis based on a combination of clinical risk factors and molecular markers, e.g., protein concentrations. These clinical risk factors and molecular markers are combined in a machine learning based algorithm which returns an output value, relating to an estimated risk of a thrombosis event in the future.

## Claims

1. An apparatus for calculating an estimation value of thrombosis risk of a patient based on patient-specific input features, said apparatus comprising:
a data interface (10, 30) for receiving said input features;
a processor for calculating said estimation value by applying a decision support algorithm as a function of numerical values derived from said received input features; and
a user interface (30) for outputting said estimation value;
wherein said input features include a combination of a plurality of clinical risk factors and a plurality of protein concentrations of said patient;
wherein the clinical risk factors include immobilization within the last three months from wearing a plaster cast, from extended bed rest at home for at least four days, or from hospitalization, surgery within the last month, a history of venous thrombosis in at least one parent, brother or sister, pregnancy or puerperium within the last three months, current use of estrogens in oral contraceptives or hormone replacement therapy, and obesity, indicated by a body mass index over 30; and
wherein the protein concentrations include a concentration of coagulation protein FVIII in blood, a concentration of coagulation protein FXI in blood, and a concentration of coagulation protein TFPI in blood.

2. The apparatus according to claim 1, wherein said processor (20) is adapted to compare said estimation value with a predetermined threshold value and to classify said estimation value based on the comparison result.

3. The apparatus according to claim 2, wherein said apparatus is adapted to allow a user to input or disable said predetermined threshold value.

4. The apparatus according to claim 1, further comprising an optimization unit (40) for applying a learning process through an optimization procedure based on a dataset stored in a database (50) so as to minimize a prediction error.

5. The apparatus according to claim 1, wherein said processor (20) is adapted to calculate a deep vein thrombosis risk score based on clinical risk factors, single nucleotide polymorphisms and protein levels.

6. A computer implemented method for calculating an estimation value of thrombosis risk of a patient based on patient-specific input features, said method comprising:
selecting said input features to include a combination of at least one clinical risk factor and a plurality of protein concentrations of said patient; and
calculating said estimation value by applying a decision support algorithm as a function of numerical values derived from said received input features;
wherein the clinical risk factors include immobilization within the last three months from wearing a plaster cast, from extended bed rest at home for at least four days, or from hospitalization, surgery within the last month, a history of venous thrombosis in at least one parent, brother or sister, pregnancy or puerperium within the last three months, current use of estrogens in oral contraceptives or hormone replacement therapy, and obesity, indicated by a body mass index over 30; and
wherein the protein concentrations include a concentration of coagulation protein FVIII in blood, a concentration of coagulation protein FXI in blood, and a concentration of coagulation protein TFPI in blood.

7. The method according to claim 6, further comprising optimizing said input features by a learning process based on a stored dataset of a plurality patients so as to minimize a prediction error.

8. The method according to claim 7, further comprising dividing said dataset into a training set, a validation set and a test set, using said training set and said validation set to select a type of machine learning function and a set of model parameters used for optimizing classifiers, using the optimized classifiers for obtaining said patient-specific input features, and using said test set for calculating said estimation value for patients of said test set based on said obtained input features.

9. A computer program product comprising program code means for causing a computer device to carry out the steps of any one of claims 6 to 8 when said computer program is run on a computer device.

## Patentansprüche

1. Vorrichtung zum Berechnen eines Schätzwerts des Thromboserisikos eines Patienten basierend auf patientenspezifischen Eingabemerkmalen, wobei die Vorrichtung umfasst:
eine Datenschnittstelle (10, 30) zum Empfangen der Eingabemerkmale;
einen Prozessor zum Berechnen des Schätzwerts durch Anwenden eines Entscheidungsunterstützungsalgorithmus als Funktion von numerischen Werten, die von den empfangenen Eingabemerkmalen abgeleitet sind; und
eine Benutzerschnittstelle (30) zum Ausgeben des Schätzwerts;
wobei die Eingabemerkmale eine Kombination aus mehreren klinischen Risikofaktoren und mehreren Proteinkonzentrationen des Patienten umfassen;
wobei die klinischen Risikofaktoren umfassen: die Immobilisierung innerhalb der letzten drei Monate nach dem Tragen eines Gipsverbandes, nach einer längeren Bettruhe zu Hause für mindestens vier Tage oder nach einem Krankenhausaufenthalt, eine Operation innerhalb des letzten Monats, eine Vorgeschichte von Venenthrombosen bei mindestens einem Elternteil, Bruder oder Schwester, Schwangerschaft oder Wochenbett innerhalb der letzten drei Monate, derzeitige Verwendung von Östrogenen in oralen Kontrazeptiva oder Hormonersatztherapien, und Fettleibigkeit, die durch einen Body-Mass-Index über 30 angezeigt wird; und
wobei die Proteinkonzentrationen eine Konzentration des Gerinnungsproteins FVIII im Blut, eine Konzentration des Gerinnungsproteins FXI im Blut und eine Konzentration des Gerinnungsproteins TFPI im Blut umfassen.

2. Vorrichtung nach Anspruch 1, wobei der Prozessor (20) angepasst ist, um den Schätzwert mit einem vorbestimmten Schwellenwert zu vergleichen, und den Schätzwert basierend auf dem Vergleichsergebnis zu klassifizieren.

3. Vorrichtung nach Anspruch 2, wobei die Vorrichtung angepasst ist, um es einem Benutzer zu ermöglichen, den vorbestimmten Schwellenwert einzugeben oder zu deaktivieren.

4. Vorrichtung nach Anspruch 1, ferner umfassend eine Optimierungseinheit (40) zum Anwenden eines Lernprozesses durch ein Optimierungsverfahren basierend auf einem in einer Datenbank (50) gespeicherten Datensatz, um einen Vorhersagefehler zu minimieren.

5. Vorrichtung nach Anspruch 1, wobei der Prozessor (20) angepasst ist, um einen Risiko-Score für tiefe Venenthrombosen basierend auf klinischen Risikofaktoren, Einzelnukleotid-Polymorphismen und Proteinspiegeln zu berechnen.

6. Computerimplementiertes Verfahren zum Berechnen eines Schätzwerts des Thromboserisikos eines Patienten basierend auf patientenspezifischen Eingabemerkmalen, wobei das Verfahren umfasst:
Auswählen der Eingabemerkmale, um eine Kombination von mindestens einem klinischen Risikofaktor und mehreren Proteinkonzentrationen des Patienten einzuschließen; und
Berechnen des Schätzwerts durch Anwenden eines Entscheidungsunterstützungsalgorithmus als Funktion von numerischen Werten, die aus den empfangenen Eingabemerkmalen abgeleitet sind;
wobei die klinischen Risikofaktoren umfassen: die Immobilisierung innerhalb der letzten drei Monate nach dem Tragen eines Gipsverbandes, nach einer längeren Bettruhe zu Hause für mindestens vier Tage oder nach einem Krankenhausaufenthalt, eine Operation innerhalb des letzten Monats, eine Vorgeschichte von Venenthrombosen bei mindestens einem Elternteil, Bruder oder Schwester, Schwangerschaft oder Wochenbett innerhalb der letzten drei Monate, derzeitige Verwendung von Östrogenen in oralen Kontrazeptiva oder Hormonersatztherapien, und Fettleibigkeit, die durch einen Body-Mass-Index über 30 angezeigt wird; und
wobei die Proteinkonzentrationen eine Konzentration des Gerinnungsproteins FVIII im Blut, eine Konzentration des Gerinnungsproteins FXI im Blut und eine Konzentration des Gerinnungsproteins TFPI im Blut umfassen.

7. Verfahren nach Anspruch 6, ferner umfassend das Optimieren der Eingabemerkmale durch einen Lernprozess basierend auf einem gespeicherten Datensatz einer Vielzahl von Patienten, um einen Vorhersagefehler zu minimieren.

8. Verfahren nach Anspruch 7, ferner umfassend das Teilen des Datensatzes in einen Trainingssatz, einen Validierungssatz und einen Testsatz, unter Verwendung des Trainingssatzes und des Validierungssatzes, um einen Typ einer maschinellen Lernfunktion und einen Satz von Modellparametern auszuwählen, die zum Optimieren von Klassifizierern verwendet werden, unter Verwendung der optimierten Klassifizierer zum Erhalten der patientenspezifischen Eingabemerkmale, und unter Verwendung des Testsatzes zum Berechnen des Schätzwerts für Patienten des Testsatzes basierend auf den erhaltenen Eingabemerkmalen.

9. Computerprogrammprodukt, umfassend Programmcodeeinrichtung, um ein Computergerät zu veranlassen, die Schritte nach einem der Ansprüche 6 bis 8 auszuführen, wenn das Computerprogramm auf einem Computergerät ausgeführt wird.

## Revendications

1. Appareil pour calculer une valeur d'estimation du risque de thrombose d'un patient sur la base de caractéristiques d'entrée spécifiques au patient, ledit appareil comprenant:
une interface de données (10, 30) pour recevoir lesdites caractéristiques d'entrée;
un processeur pour calculer ladite valeur d'estimation en appliquant un algorithme d'aide à la décision en fonction de valeurs numériques dérivées desdites caractéristiques d'entrée reçues; et
une interface utilisateur (30) pour sortir ladite valeur d'estimation;
où lesdites caractéristiques d'entrée comprennent une combinaison d'une pluralité de facteurs de risque clinique et d'une pluralité de concentrations en protéines dudit patient;
où les facteurs de risque clinique comprennent l'immobilisation au cours des trois derniers mois de porter un plâtre, d'un alitement prolongé à la maison pendant au moins quatre jours, ou d'une hospitalisation, une intervention chirurgicale au cours du dernier mois, une histoire de thrombose veineuse chez au moins un parent, frère ou sœur, une grossesse ou puerpéralité au cours des trois derniers mois, une utilisation actuelle d'œstrogènes dans des contraceptifs oraux ou dans une hormonothérapie substitutive, et obésité, indiquée par un indice de masse corporelle supérieur à 30; et
où les concentrations de protéines comprennent une concentration de protéine de coagulation FVIII dans le sang, une concentration de protéine de coagulation FXI dans le sang, et une concentration de protéine de coagulation TFPI dans le sang.

2. Appareil selon la revendication 1, dans lequel ledit processeur (20) est adapté pour comparer ladite valeur d'estimation à une valeur de seuil prédéterminée et pour classer ladite valeur d'estimation sur la base du résultat de comparaison.

3. Appareil selon la revendication 2, dans lequel ledit appareil est adapté pour permettre à un utilisateur d'entrer ou de désactiver ladite valeur de seuil prédéterminée.

4. Appareil selon la revendication 1, comprenant en outre une unité d'optimisation (40) pour appliquer un processus d'apprentissage à travers une procédure d'optimisation sur la base d'un ensemble de données stocké dans une base de données (50) afin de minimiser une erreur de prédiction.

5. Appareil selon la revendication 1, dans lequel ledit processeur (20) est adapté pour calculer un score de risque de thrombose veineuse profonde sur la base de facteurs de risque clinique, de polymorphismes mononucléotidiques et de niveaux de protéines.

6. Procédé mis en œuvre par ordinateur pour calculer une valeur d'estimation du risque de thrombose d'un patient sur la base de caractéristiques d'entrée spécifiques au patient, ladite procédé comprenant:
sélectionner lesdites caractéristiques d'entrée pour inclure une combinaison d'au moins un facteur de risque clinique et une pluralité de concentrations de protéines dudit patient; et
calculer ladite valeur d'estimation en appliquant un algorithme d'aide à la décision en fonction de valeurs numériques dérivées desdites caractéristiques d'entrée reçues;
où les facteurs de risque clinique comprennent l'immobilisation au cours des trois derniers mois de porter un plâtre, d'un alitement prolongé à la maison pendant au moins quatre jours, ou d'une hospitalisation, une intervention chirurgicale au cours du dernier mois, une histoire de thrombose veineuse chez au moins un parent, frère ou sœur, une grossesse ou puerpéralité au cours des trois derniers mois, une utilisation actuelle d'œstrogènes dans des contraceptifs oraux ou dans une hormonothérapie substitutive, et obésité, indiquée par un indice de masse corporelle supérieur à 30; et
où les concentrations de protéines comprennent une concentration de protéine de coagulation FVIII dans le sang, une concentration de protéine de coagulation FXI dans le sang, et une concentration de protéine de coagulation TFPI dans le sang.

7. Procédé selon la revendication 6, comprenant en outre l'optimisation desdites caractéristiques d'entrée par un processus d'apprentissage basé sur un ensemble de données stocké d'une pluralité de patients afin de minimiser une erreur de prédiction.

8. Procédé selon la revendication 7, comprenant en outre la division dudit ensemble de données en un ensemble de formation, un ensemble de validation et un ensemble de test, utilisant ledit ensemble de formation et ledit ensemble de validation pour sélectionner un type de fonction d'apprentissage automatique et un ensemble de paramètres de modèle utilisés pour optimiser des classificateurs, utilisant les classeurs optimisés pour obtenir lesdites caractéristiques d'entrée spécifiques au patient, et utilisant ledit ensemble de test pour calculer ladite valeur d'estimation pour les patients dudit ensemble de test sur la base desdites caractéristiques d'entrée obtenues.

9. Produit de programme informatique comprenant un moyen de code de programme pour amener un dispositif informatique à exécuter les étapes selon l'une quelconque des revendications 6 à 8 lorsque ledit programme informatique est exécuté sur un dispositif informatique.
